# EUROPÄISCHE PATENTANMELDUNG

(11) **EP 1 297 817 A2**
(43) Veröffentlichungstag der Anmeldung: **02.04.2003**
(21) Anmeldenummer: 02021772.5
(22) Anmeldetag: 26.09.2002
(51) Int. Cl.: A61K 7/13

(54) **Verfahren zum oxidativen Färben von menschlichen Haaren**

(30) Priorität: 26.09.2001 DE 10147575
(71) Anmelder: KPSS-Kao Professional Salon Services GmbH, 64297 Darmstadt (DE)
(72) Erfinder: Lorenz, Heribert, 64401 Gross-Bieberau (DE)

(57) **Zusammenfassung**

Ein neues Verfahren zum Färben von menschlichen Haaren, das insbesondere Färbungen mit einer verstärkten Farbintensität ermöglicht, besteht darin, daß eine mindestens eine Entwicklersubstanz sowie ein Oxidationsmittel enthaltende Oxidationsfarbstoffmischung auf das Haar aufgebracht, und, nach erfolgter Farbstoffeinwirkung, aus dem Haar ausgespült wird, wobei diese Oxidationsfarbstoffmischung Dihydroxyaceton und/oder Alloxan und/oder Methylglyoxal und/oder Kaliumjodid enthält.

## Beschreibung

Die vorliegende Erfindung betrifft ein neues Verfahren zum oxidativen Färben von menschlichen Kopfhaaren, das eine verbesserte Deckkraft und Farbintensität bzw. -stabilität der Färbung bewirkt.

Die nach wie vor übliche Art der Haarfärbung ist die Färbung mit Oxidationsfarbstoffen, die unmittelbar vor der Anwendung mit Oxidationsmittel-Zusammensetzungen, insbesondere auf Basis von verdünntem Wasserstoffperoxid, vermischt und auf das Haar aufgetragen werden.

In manchen Fällen, z.B. bei Einstellung spezieller Farbnuancen, läßt allerdings die Intensität und Stabilität der mit diesen Zusammensetzungen erzielten Haarfärbungen etwas zu wünschen übrig.

Die Erfindung hat sich die Aufgabe gestellt, diese Nachteile zu überwinden.

Die Lösung dieser Aufgabe besteht in der Anwendung eines neuen Verfahrens zum oxidativen Färben menschlicher Haare, das aus den folgenden Verfahrensschritten besteht:

Aufbringen einer, mindestens eine Entwicklersubstanz sowie vorzugsweise auch mindestens eine Kupplersubstanz sowie ein Oxidationsmittel enthaltenden Oxidationsfarbstoffmischung in an sich bekannter Weise auf das Haar; anschließend, vorzugsweise nach etwa fünfzehn- bis etwa dreißigminütiger Einwirkung, Spülen des Haares nach erfolgter Farbstoffeinwirkung und Trocknung desselben, wobei die aufgebrachte Farbstoffmischung Dihydroxyaceton und/oder Alloxan und/oder Methylglyoxal und/oder Kaliumjodid enthält.

Durch die Anwendung dieses Verfahrens wird, bedingt durch die Anwesenheit des Dihydroxyacetons und/oder Alloxans und/oder Methylglyoxals und/oder Kaliumjodids, eine ausgezeichnete Farbintensität erreicht.

Die zum Einsatz gelangenden Oxidationsfarbstoffmischungen, die mindestens eine Entwickler- und vorzugsweise mindestens auch eine Kupplersubstanz sowie ein Oxidationsmittel enthalten, sind an sich bereits bekannt.

Es wird hierzu auf den Stand der Technik, z.B. auf die Monographie von K. Schrader, "Grundlagen und Rezepturen der Kosmetika", 2. Aufl., S. 784-804 (1989), verwiesen; die dort beschriebenen Produkte sind im Rahmen des erfindungsgemäßen Verfahrens ebenso einsetzbar wie die aus dem umfangreichen Stand der Technik bekannten weiteren Entwickler- und Kupplersubstanzen sowie Nuanceure.

Beispielhafte Entwicklersubstanzen sind insbesondere p-Phenylendiamine und deren Derivate wie 1,4-Diaminobenzol, 2,5-Diaminotoluol, 1,2,4-Triaminobenzol, 1,4-Diamino-2-chlorbenzol, 2-(2,5-Diaminophenyl)ethanol, 4-Aminodiphenylamin, 4,4'-Diaminodophenylamin, 2-(2-Hydroxyethylamino)-5-aminotoluol und 1-Amino-4-bis-(2'-hydroxyethyl)-aminobenzol bzw. deren wasserlösliche Salze; Tetraaminopyrimidine, Triaminohydroxypyrimidine, Pyrazol und dessen Derivate wie z.B. 4,5-Diaminopyrazol und deren Salze; 4-Aminophenole und deren Substitutionsprodukte wie 4-Amino-3-methylphenol, und vorzugsweise substituierte Indole oder Indoline (vgl. DE 37 39 028 A1 und DE 41 39 675 A1), beispielsweise 5-Hydroxyindol, 5,6-Dihydroxyindol oder 6-Hydroxyindolin bzw. 5,6-Dihydroxyindolin. Im Prinzip sind alle Entwicklersubstanzen für diesen Zweck geeignet.

Der Zusatz des Dihydroxyacetons und/oder Alloxans und/oder Methylglyoxals und/oder Kaliumjodids kann unmittelbar vor dem Vermischen des Oxidationsfarbstoffvorproduktes mit dem Peroxid erfolgen, wobei sichergestellt sein sollte, daß in der applikationsfertigen Mischung mindestens 0,0001, vorzugsweise 0,5 Gew.-% derselben an Dihydroxyaceton und/oder Alloxan und/oder Methylglyoxal und/oder Kaliumjodid vorliegen. Die Höchstmenge an Dihydroxyaceton und/oder Alloxan und/oder Methylglyoxal und/oder Kaliumjodid in der Mischung liegt bei etwa 5, vorzugsweise etwa 2,5 Gew.-%.

Obwohl diese Substanz im Prinzip auch separat dem Reaktionsgemisch zugesetzt werden kann (beispielsweise als Pulver, Tablette, Lösung, Gel, etc.), ist es zweckmäßig, diese entweder der Oxidationsfarbstoffzusammensetzung (bevorzugt) oder der Peroxidzusammensetzung zuzusetzen.
Beispielhafte Kupplersubstanzen sind Resorcin, 2-Methylresorcin, 4-Chlorresorcin, 2-Amino-4-chlorphenol, 4-(N-methyl)-aminophenol, 2-Aminophenol, 3-Aminophenol, 1-Methyl-2-hydroxy-4-aminobenzol, 3-N,N-Dimethylaminophenol, 5-Amino-2-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'hydroxyethyl)amino]benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,7-Dihydroxynaphthalin, 2,7-Dihydroxynaphthalin, 4-Hydroxy-1,2-methylendioxybenzol, 2,4-Diamino-3-chlorphenol und/oder 1-Methoxy-2-amino-4-(2'-hydroxyethylamino)benzol bzw. deren wasserlösliche Salze, ohne daß diese beispielhafte Aufzählung Anspruch auf Vollständigkeit erheben könnte.
Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbzusammensetzungen kann insgesamt etwa 0,1 bis etwa 5 Gew.-%, je nach gewünschter Färbung, betragen.

Als Oxidationsmittel werden vor allem verdünnte Wasserstoffperoxid-Lösungen, -Emulsionen oder -Gele eingesetzt, möglich, aber weniger üblich ist auch die Verwendung weiterer Peroxide wie Erdalkaliperoxide, Harnstoffperoxid, Melaminperoxid, etc. in entsprechenden stöchiometrischen Mengen.
Auch eine Luftoxidation ist prinzipiell möglich.

Die Oxidationsfarbstoffzusammensetzungen können als Lösungen, Cremes, Pasten, Gele, Aerosole, etc. Verwendung finden.

Die Einwirkungszeit liegt vorzugsweise bei etwa zehn bis dreißig, insbesondere bei jeweils etwa 15 bis 25 Minuten. Das erfindungsgemäße Verfahren eignet sich dabei sowohl zum ganzheitlichen, d.h. erstmaligen Färben der Haare als auch zum Nachfärben.

Der pH-Wert der gebrauchsfertigen Zusammensetzung liegt dabei sowohl im alkalischen, d.h., bei etwa 7,1 bis 11, im neutralen oder schwach sauren, d.h., bei etwa 5 bis 6,9, Bereich.

In den folgenden Beispielen wird die Erfindung illustriert.

### Beispiel 1

Auf Strähnen gebleichten Menschenhaares wurde eine Färbezusammensetzung, die durch Vermischen von gleichen Gewichtsteilen einer 6%-igen Wasserstoffperoxidlösung und eines Oxidationsfarbstoffvorprodukts der folgenden Zusammensetzung erhalten wurde, und einen pH-Wert von 9,8 aufwies, aufgebracht.

| Trägermasse | |
|---|---|
| Cetylstearylalkohol | 11,00 (Gew.%) |
| Oleth-5 | 5,00 |
| Ölsäure | 2,50 |
| Stearinsäuremonoethanolamid | 2,50 |
| Cocosfettsäuremonoethanolamid | 2,50 |
| Natriumlaurylsulfat | 1,70 |
| Natriumsulfit | 1,00 |
| 1,2-Propandiol | 1,00 |
| Ascorbinsäure | 0,50 |
| Ammoniumchlorid | 0,50 |
| EDTA, Tetranatriumsalz | 0,20 |
| Parfum | 0,40 |
| Weizenproteinhydrolysat | 0,20 |
| Silica | 0,10 |

| Oxidationsfarbstoffmischung und Wirkstoff | |
|---|---|
| 2,5,6-Triamino-4-hydroxypyrimidinsulfat | 0,01 |
| 2,5-Diaminotoluolsulfat | 0,55 |
| 4-Chlorresorcin | 0,17 |
| Resorcin | 0,05 |
| 3-Aminophenol | 0,03 |
| Methylglyoxal | 0,50 |
| Wasser | ad 100,00 |

Nach 20-minütiger Einwirkung wurde mit Wasser gut ausgespült und getrocknet.

Es wurde eine starke, intensive, ausdrucksvolle Mittelblondfärbung erhalten, die auch nach acht Haarwäschen unverändert war.

Strähnen, die in gleicher Weise 20 Minuten mit einer Farbzusammensetzung behandelt worden waren, die kein Methylglyoxal enthielt, zeigten demgegenüber eine schwächere Färbung, die nach fünf Haarwäschen deutlich blasser war als die nach dem erfindungsgemäßen Verfahren erzielte.

### Beispiel 2

In eine der Zusammensetzung nach Beispiel 1 entsprechende Trägermasse wurde die folgende Oxidationsfarbstoffmischung eingebracht:

| | |
|---|---|
| 2,5,6-Triamino-4-hydroxypyrimidinsulfat | 0,01 (Gew.-%) |
| 2,5-Diaminotoluolsulfat | 0,90 |
| 2-Amino-3-hydroxypyridin | 0,80 |
| 1-Naphthol | 0,17 |
| 3-Aminophenol | 0,10 |
| Resorcin | 0,03 |
| Alloxan | 0,50 |

Dieses Produkt wurde mit 6%-iger H₂O₂-Lösung im Gewichtsverhältnis 1:1 vermischt (pH 9,5) und auf Strähnen aus gebleichtem Menschenhaar aufgebracht.

Nach 25-minütiger Einwirkung wurde, mit Wasser ausgewaschen, shampooniert und getrocknet.

Es wurde eine intensive, glänzende Mahagoni-Färbung erhalten, die auch nach acht Haarwäschen noch stabil war.

Die gleiche Behandlung über 25 Minuten mit einer identischen Farbzusammensetzung ohne Alloxan führte zu wesentlich schwächeren Ausfärbungen.

### Beispiel 3

In eine der Zusammensetzung nach Beispiel 1 entsprechende Trägermasse wurde die folgende Oxidationsfarbstoffmischung eingebracht:

| | |
|---|---|
| 2,5-Diaminotoluolsulfat | 0,80 (Gew.-%) |
| Resorcin | 0,30 |
| 2-Amino-3-hydroxypyridin | 0,06 |
| 3-Aminophenol | 0,03 |
| Alloxan | 0,50 |
| Kaliumjodid | 0,002 |

Dieses Produkt wurde mit 6%-iger H₂O₂-Lösung im Gewichtsverhältnis 1:1 gemischt (pH 9,6) und auf Strähnen aus gebleichtem Menschenhaar aufgebracht.
Nach 20-minütiger Einwirkung wurde gründlich ausgespült und getrocknet.
Es wurde eine intensive rotbraune Färbung erhalten, die auch nach acht Haarwäschen noch stabil war.
Weglassen der Wirkstoffe führte zu einer wesentlich blasseren Färbung.

### Beispiel 4

In eine Trägermasse nach Beispiel 1, deren pH-Wert so eingestellt war, daß die gebrauchsfertige Mischung einen pH-Wert von 6,8 ergab, wurde die folgende Oxidationsfarbstoffmischung eingebracht:

| | |
|---|---|
| 1-(β-Hydroxyethyl)-2,5-diaminobenzolsulfat | 0,28 (Gew.-%) |
| 4-Amino-3-methylphenol | 0,14 |
| 5-Amino-2-methylphenol | 0,28 |

Diese Zusammensetzung wurde mit der folgenden 6%-igen H₂O₂-Lotion Rezeptur im Gewichtsverhältnis 1:1 gemischt, wobei ein pH-Wert von 6,8 erreicht wurde, und auf Strähnen aus gebleichtem Menschenhaar aufgebracht.

| | |
|---|---|
| Wasserstoffperoxid | 6,0 (Gew.-%) |
| Cetearylalkohol | 1,8 |
| Phosphorsäure | 0,4 |
| Natriumlaurylsulfat | 0,2 |
| Dinatriumphosphat | 0,1 |
| Salicylsäure | 0,1 |
| Dihydroxyaceton | 0,30 |
| Methylglyoxal | 0,30 |
| Wasser | ad 100,0 |

Nach zwanzigminütiger Einwirkung wurde gewaschen und getrocknet.

Es wurde eine sehr intensive, kräftige rotviolette Ausfärbung erhalten. Wiederholung der Ausfärbung mit einer Peroxid-Zusammensetzung, die keine Wirkstoffe enthielt, führte zu einer Rosaviolett-Färbung.

### Beispiel 5

In die Basisrezeptur nach Beispiel 1 wurde die folgende Oxidationsfarbstoffzusammensetzung eingebracht:

| | |
|---|---|
| 1-Hydroxyethyl-4,5-diaminopyrazolsulfat | 0,54 (Gew.-%) |
| 4-Amino-2-hydroxytoluol | 0,27 |

Das Produkt wurde mit der 6%-igen H₂O₂-Lotion aus Beispiel 4 im Gewichtsverhältnis 1:1, wobei ein pH-Wert von 9,8 erreicht wurde, vermischt und auf das Haar aufgebracht. Nach zwanzigminütiger Einwirkung wurde gewaschen und getrocknet, wobei eine glänzend-intensive Rotkupfer-Färbung erreicht wurde.

Wiederholung der Ausfärbung mit einer Peroxid-Zusammensetzung, die keine Wirkstoffe enthielt, führte zu einer deutlich blasseren Rot-Färbung.

### Beispiel 6

| | | |
|---|---|---|
| a) | 5,6-Dihydroxyindol | 0,50 (Gew.-%) |
| | Dihydroxyaceton | 0,50 |
| | Kaliumjodid | 0,002 |
| | Cetearylalkohol | 10,00 |
| | Ceteareth-20 | 10,00 |
| | Kokosfettalkohol | 5,00 |
| | Ascorbinsäure | 0,50 |
| | Ammoniumhydroxid | ad pH 8,0 |
| | Wasser | ad 100,00 |

Diese Zusammensetzung wurde sowohl auf Woll-Läppchen als auch auf angefeuchtetes, gebleichtes Menschenhaar aufgebracht.
Nach dreißigminütiger Luftoxidation wurde gewaschen und getrocknet.
Es wurde ein glänzende, intensive mittelaschblonde Färbung erhalten.

Die Färbung mit einer identischen Zusammensetzung ohne Wirkstoffe führte zu einem deutlich blasseren Aschblondfarbton.

### b)

Auf gleiche Weise wie unter a) beschrieben wurde mit der folgenden Farbzusammensetzung für 20 Minuten gefärbt.

| | |
|---|---|
| 5,6-Dihydroxyindolinhydrobromid | 1,00 (Gew.-%) |
| Alloxan | 1,00 |
| Kaliumjodid | 0,005 |
| Cetearylalkohol | 10,00 |
| Ceteareth-20 | 10,00 |
| Kokosfettalkohol | 5,00 |
| Ascorbinsäure | 0,50 |
| Ammoniumhydroxid | ad pH 9,0 |
| Wasser | ad 100,00 |

Es wurde eine intensive, glänzende hellblonde Färbung erhalten, während das Weglassen der Wirkstoffe lediglich eine helle aschblonde Farbe ergab.

## Patentansprüche

1. Verfahren zum Färben von menschlichen Haaren durch Aufbringen einer Oxidationsfarbstoffmischung, enthaltend mindestens eine Entwicklersubstanz und mindestens ein Oxidationsmittel, auf das Haar und Ausspülen und Trocknen des Haares nach erfolgter Einwirkung in an sich bekannter Weise, **dadurch gekennzeichnet, daß** die Oxidationsfarbstoffmischung Dihydroxyaceton und/oder Alloxan und/oder Methylglyoxal und/oder Kaliumjodid enthält.

2. Verfahren nach Anspruch 1, **dadurch gekennzeichnet, daß** die Oxidationsfarbstoffmischung 0,0001 bis 5 Gew.-%, berechnet auf die Gesamtzusammensetzung, Dihydroxyaceton und/oder Alloxan und/oder Methylglyoxal und/oder Kaliumjodid enthält.

3. Verfahren nach Anspruch 1 und/oder 2, **dadurch gekennzeichnet, daß** die Oxidationsfarbstoffmischung mindestens eine Kupplersubstanz enthält.

4. Verwendung von Dihydroxyaceton und/oder Alloxan und/oder Methylglyoxal und/oder Kaliumjodid in Haarfärbemitteln auf Basis mindestens eines Oxidationsfarbstoffvorprodukts.
